# EUROPÄISCHE PATENTSCHRIFT

(11) **EP 3 234 229 B1**
(45) Veröffentlichungstag und Bekanntmachung des Hinweises auf die Patenterteilung: **01.04.2020**
(21) Anmeldenummer: 15823161.3
(22) Anmeldetag: 23.12.2015
(51) Int. Cl.: C25B 15/08, C25B 1/00, C25B 3/04

(54) **VERFAHREN UND ELEKTROLYSESYSTEM ZUR KOHLENSTOFFDIOXID-VERWERTUNG**
METHOD AND ELECTROLYSIS SYSTEM FOR UTILIZING CARBON DIOXIDE
PROCÉDÉ ET SYSTÈME D'ÉLECTROLYSE POUR LE RECYCLAGE DU DIOXYDE DE CARBONE

(30) Priorität: 06.02.2015 DE 102015202117
(43) Veröffentlichungstag der Anmeldung: 25.10.2017
(73) Patentinhaber: Siemens Aktiengesellschaft, 80333 München (DE)
(72) Erfinder: KRAUSE, Ralf, 91074 Herzogenaurach (DE); RELLER, Christian, 32425 Minden (DE); SCHMID, Günter, 91334 Hemhofen (DE); VOLKOVA, Elena, 91052 Erlangen (DE)
(86) Internationale Anmeldenummer: PCT/EP2015/081145
(87) Internationale Veröffentlichungsnummer: WO 2016/124300

(56) Entgegenhaltungen:
- EP-A1- 2 832 421
- AU-A- 6 811 981
- US-A1- 2012 228 147
- JOHN-PAUL JONES ET AL: "Electrochemical CO2 Reduction: Recent Advances and Current Trends", ISRAEL JOURNAL OF CHEMISTRY., Bd. 54, Nr. 10, 9. September 2014 (2014-09-09), Seiten 1451-1466, XP055253468, IL ISSN: 0021-2148, DOI: 10.1002/ijch.201400081

## Beschreibung

Die vorliegende Erfindung betrifft ein Verfahren und ein Elektrolysesystem zur Kohlenstoffdioxid-Verwertung und Kohlenstoffmonoxid-Herstellung. In einem Elektrolyseschritt wird Kohlenstoffdioxid in eine Elektrolysezelle eingeleitet und an einer Kathode reduziert.

### Stand der Technik

Aktuell wird ca. 80 % des weltweiten Energiebedarfs durch die Verbrennung von fossilen Brennstoffen gedeckt, deren Verbrennungsprozesse eine weltweite Emission von etwa 34000 Millionen Tonnen Kohlenstoffdioxid in die Atmosphäre pro Jahr verursacht. Durch diese Freisetzung in die Atmosphäre wird der Großteil an Kohlenstoffdioxid entsorgt, was z.B. bei einem Braunkohlekraftwerk bis zu 50000 Tonnen pro Tag betragen kann. Kohlenstoffdioxid gehört zu den sogenannten Treibhausgasen, deren negative Auswirkungen auf die Atmosphäre und das Klima diskutiert werden. Da Kohlenstoffdioxid thermodynamisch sehr niedrig liegt, kann es nur schwierig zu wiederverwertbaren Produkten reduziert werden, was die tatsächliche Wiederverwertung von Kohlenstoffdioxid bisher in der Theorie beziehungsweise in der akademischen Welt belassen hat.

Ein natürlicher Kohlenstoffdioxid-Abbau erfolgt beispielsweise durch Fotosynthese. Dabei werden in einem zeitlich und auf molekularer Ebene räumlich in viele Teilschritte aufgegliederten Prozess Kohlenstoffdioxid zu Kohlehydraten umgesetzt. Dieser Prozess ist so nicht einfach großtechnisch adaptierbar. Eine Kopie des natürlichen Fotosyntheseprozesses mit großtechnischer Fotokatalyse ist nicht ausreichend effizient.

Eine Alternative stellt die elektrochemische Reduktion des Kohlenstoffdioxids dar. Systematische Untersuchungen der elektrochemischen Reduktion von Kohlenstoffdioxid sind noch ein relativ junges Entwicklungsfeld. Erst seit wenigen Jahren gibt es Bemühungen, ein elektrochemisches System zu entwickeln, das eine akzeptable Kohlenstoffdioxidmenge reduzieren kann. Forschungen im Labormaßstab haben gezeigt, dass zur Elektrolyse von Kohlenstoffdioxid bevorzugt Metalle als Katalysatoren einzusetzen sind. Aus der Veröffentlichung Electrochemical CO₂ reduction on metal electrodes von Y. Hori, veröffentlicht in: C. Vayenas, et al. (Eds.), Modern Aspects of Electrochemistry, Springer, New York, 2008, pp. 89-189, sind Faraday Effizienzen an unterschiedlichen Metallkathoden zu entnehmen, siehe Tabelle 1 . Wird Kohlenstoffdioxid beispielsweise an Silber-, Gold-, Zink-, Palladium- und Galliumkathoden nahezu ausschließlich zu Kohlenstoffmonoxid reduziert, entstehen an einer Kupferkathode, eine Vielzahl an Kohlenwasserstoffen als Reaktionsprodukte.

So würden beispielsweise an einer Silberkathode überwiegend Kohlenstoffmonoxid und wenig Wasserstoff entstehen. Die Reaktionen an Anode und Kathode können mit folgenden Reaktionsgleichungen dargestellt werden:

| | |
|---|---|
| Kathode: | 2 CO₂ + 4 e⁻ + 4 H⁺ → 2 CO + 2 H₂O |
| Anode: | 2 H₂O → O₂ + 4 H⁺ + 4 e⁻ |

Von besonderem wirtschaftlichem Interesse ist beispielsweise die elektrochemische Erzeugung von Kohlenstoffmonoxid, Methan oder Ethen. Dabei handelt es sich um energetisch höherwertige Produkte als Kohlenstoffdioxid.

**Tabelle 1: In der Tabelle sind Faraday Effizienzen [%] von Produkten angegeben, die bei der Kohlenstoffdioxid-Reduktion an verschiedenen Metallelektroden entstehen. Die angegebenen Werte gelten für eine 0,1 M Kaliumhydrogencarbonatlösung als Elektrolyten, und Stromdichten unterhalb von 10mA/cm².**

| Elektrode | CH₄ | C₂H₄ | C₂H₅OH | C₃H₇OH | CO | HCOO⁻ | H₂ | Total |
|---|---|---|---|---|---|---|---|---|
| Cu | 33.3 | 25.5 | 5.7 | 3.0 | 1.3 | 9.4 | 20.5 | 103.5 |
| Au | 0.0 | 0.0 | 0.0 | 0.0 | 87.1 | 0.7 | 10.2 | 98.0 |
| Ag | 0.0 | 0.0 | 0.0 | 0.0 | 81.5 | 0.8 | 12.4 | 94.6 |
| Zn | 0.0 | 0.0 | 0.0 | 0.0 | 79.4 | 6.1 | 9.9 | 95.4 |
| Pd | 2.9 | 0.0 | 0.0 | 0.0 | 28.3 | 2.8 | 26.2 | 60.2 |
| Ga | 0.0 | 0.0 | 0.0 | 0.0 | 23.2 | 0.0 | 79.0 | 102.0 |
| Pb | 0.0 | 0.0 | 0.0 | 0.0 | 0.0 | 97.4 | 5.0 | 102.4 |
| Hg | 0.0 | 0.0 | 0.0 | 0.0 | 0.0 | 99.5 | 0.0 | 99.5 |
| In | 0.0 | 0.0 | 0.0 | 0.0 | 2.1 | 94.9 | 3.3 | 100.3 |
| Sn | 0.0 | 0.0 | 0.0 | 0.0 | 7.1 | 88.4 | 4.6 | 100.1 |
| Cd | 1.3 | 0.0 | 0.0 | 0.0 | 13.9 | 78.4 | 9.4 | 103.0 |
| Tl | 0.0 | 0.0 | 0.0 | 0.0 | 0.0 | 95.1 | 6.2 | 101.3 |
| Ni | 1.8 | 0.1 | 0.0 | 0.0 | 0.0 | 1.4 | 88.9 | 92.4 |
| Fe | 0.0 | 0.0 | 0.0 | 0.0 | 0.0 | 0.0 | 94.8 | 94.8 |
| Pt | 0.0 | 0.0 | 0.0 | 0.0 | 0.0 | 0.1 | 95.7 | 95.8 |
| Ti | 0.0 | 0.0 | 0.0 | 0.0 | 0.0 | 0.0 | 99.7 | 99.7 |

Nun tritt bei allen elektrochemischen Reduktionsverfahren für die Konversion von Kohlenstoffmonoxid oder Kohlenstoffdioxid je nach den Prozessbedingungen ein, häufig unerwünschter, Nebeneffekt auf: Es entstehen Formiate, die Salze der Ameisensäure, welche die Zusetzung freier Oberflächen mit Kohlenstoffablagerungen begünstigen. Die Elektroden- bzw. Katalysatorfläche in einer Elektrolysezelle würde dadurch deaktiviert, denn die Bildung von Graphitablagerungen führt zu einer Verkleinerung der katalytisch aktiven Fläche. Im Falle der Kohlenstoffdioxidreduktion würde zum einen der Umsatz vermindert sowie auch die Selektivität des Katalysators gegenüber Kohlenwasserstoffen negativ beeinflusst. Dies bedeutet gleichermaßen Einbußen in der Langzeitstabilität der Elektroden und der Wirtschaftlichkeit des Verfahrens.

Die Problematik einer Katalysatorverkokung, also der Zersetzung des Katalysators mit Graphit, ist bisher bereits aus anderen Themengebieten bekannt, die die Umsetzung von Kohlenstoffmonoxid oder Kohlenstoffdioxid betreffen. Beispielsweise erfolgt auch beim katalytischen Crackverfahren eine Katalysatorverkokung. Die Regenerierung der Elektroden erfolgt dabei nicht in situ, sondern diese müssen dazu ausgebaut werden. Dann werden die Kohlenstoffablagerungen mit Hilfe von Sauerstoff bei Temperaturen von ca. 700°C abgebrannt. Als Beispiel sei das industriell eingesetzte FCC-Verfahren (fluid catalytic cracking) genannt, welches auf dem Reaktor-Generatorprinzip beruht.

Eine alternative Herangehensweise um dem Problem der Katalysatorverkokung zu begegnen, ist aus dem Themengebiet der Fischer-Tropsch-Katalyse bzw. bei Methanisierungsreaktionen bekannt. Dabei wird der Katalysatordeaktivierung durch Koksablagerungen während dem Prozess entgegengewirkt. Der Start der Bildung von Kohlenstoffablagerungen auf einheitlichen Metallflächen findet nämlich vorzugsweise statt, wenn das Verhältnis der Partialdrücke von Kohlenstoffmonoxid zu Wasserstoff größer als 2 ist, bei einer Temperatur unter 673 K oder größer als 1 ist bei einer Temperatur größer als 673 K. Dies kann bspw. der Veröffentlichung von M.T. Tavares, I. Alstrup, C.A.A. Bernardo, erschienen in: Materials and Corrosion 1999, 50, 681-685 entnommen werden. Dementsprechend kann durch einen Wasserstoffüberschuss die Katalysatorverkokung beim Sabatier-Prozess unterdrückt werden. Auch diese Methode liegt in einem zu hohen Temperaturbereich als dass sie in situ erfolgen könnte. Deshalb sind diese Varianten in einem kontinuierlichen Elektrolyseverfahren nicht anwendbar.

Eine weitere alternative Variante deaktivierte Elektrodenoberflächen zu regenerieren ist die Reaktivierung durch Umpolung des Potentials. Die Elektrode, die als Kathode fungiert, wird so umgepolt, dass das an ihr anliegende Potential im oxidativen Bereich liegt. Dabei wird natürlich auch die Elektrode selbst oxidiert und dementsprechend können Metallionen in Lösung gehen. Dieses Regenerierungsverfahren ist beispielsweise in der Veröffentlichung von Y.Yano, S. Yamasaki, J. Appl. Electrochem. 2008, 38, 1721-1726, beschrieben. Die Nachteile dieser Methode liegen zum einen in dem hohen Energieverlust durch die häufig notwendige Umpolung, zum anderen in den unerwünschten Ausfallzeiten des Systems.

Katalytische Konversionsmethoden zur Spaltung von Formiaten sind bisher außerdem beispielsweise aus der chemischen Wasserstoffspeicherung bekannt (Boddien, Albert; Mellmann Dörthe; Gärtner, Felix; Jackstell, Ralf; Junge, Henrik; Dyson, Paul J.; Laurency, Gábor; Ludwig, Ralf; Beller, Matthias, Science, Volume 333, Issue 6050, pp. 1733-1736 (2011)). Des Weiteren wurde die thermische Zersetzung von Übergangsmetall-Formiaten untersucht (Rienäcker, W. Toursel, Zeitschrift ft für anorganische und allgemeine Chemie, 1961, 307, 235-254). Außerdem ist die katalytische Wirkung speziell von Ruthenium-Komplexen auf die Umsetzung von Formiaten bekannt (C. Fellay, Paul J.D., G. Laurenczy, Angewandte Chemie, 2008, 120, 4030-4032; Weijia Gan, Paul J. Dyson, Gábor Laurenczy, Reaction Kinetics and Catalysis Letters 2009, 98, 205-213.). Heterogen katalysierte Umsetzungen von Formiaten bedienen sich bisher stets eines Edelmetalls oder einer Oberflächenmodifikation mit einem Edelmetall (Nan Yi, H. Saltsburg, M.F. Stephanopoulos, 2013, 6, 816-819; Xiaojun Gu, Zhang-Hui Lu, Hai-Long Jiang, Tomoki Akita, Qiang Xu, J. Am. Chem. Soc., 2011, 133 (31), 11822-11825; Seung-Young Chung, Sung-Hyun Uhm, Jae-Kwang Lee, Sung-Jin Kang, Yong-Sug Tak, and Jae-Young Lee, J. Ind. Eng. Chem., Vol. 13, No. 3, (2007) 339-344).

Die AU-A1-68 119/81 zeigt ein Verfahren zur Umwandlung von Sonnenenergie, umfassend Umwandeln der Sonnenenergie in elektrischen Strom, wobei der so erhaltene Strom zum Elektrolysieren von Kohlendioxid und Wasser zur Herstellung von Ameisensäure und Zersetzen der Ameisensäure in Kohlenmonoxid und Wasser verwendet wird.

Ferner zeigt die US 2012/0228147 A1 ein Verfahren und Systeme zur elektrochemischen Herstellung von Ameisensäure. Dabei beinhaltet das Verfahren Schritte (A) bis (D). Im Schritt (A) wird Wasser in eine erste Kammer einer elektrochemischen Zelle eingeleitet. Im Schritt (B) wird Kohlendioxid in eine zweite Kammer der elektrochemischen Zelle einbracht. Im Schritt (C) wird ein elektrisches Potential zwischen der Anode und der Kathode in der elektrochemischen Zelle angelegt, das ausreicht, um das Kohlendioxid zu Ameisensäure zu reduzieren. Im Schritt (D) wird eine Konzentration von Ameisensäure in der zweiten Kammer bei oder unter etwa 500 ppm gehalten.

Folglich stellt es sich als technisch erforderlich dar, eine verbesserte Lösung für die Kohlenstoffdioxid-Verwertung vorzuschlagen, welche die aus dem Stand der Technik bekannten Nachteile vermeidet. Insbesondere soll die vorzuschlagende Lösung nicht nur einen effektiven Kohlenstoffdioxidabbau ermöglichen sondern auch eine wirtschaftliche, langlebige Kohlenstoffdioxid-Verwertung gewährleisten. Es ist Aufgabe der Erfindung, ein Verfahren und ein System zur elektrochemischen Kohlenstoffdioxid-Verwertung unter Vermeidung einer Verkokung anzugeben.

Diese der vorliegenden Erfindung zugrundeliegenden Aufgaben werden durch ein Verfahren gemäß dem Patentanspruch 1 durch ein Elektrolysesystem gemäß dem Patentanspruch 3 gelöst. Vorteilhafte Ausgestaltungen der Erfindung sind Gegenstand der Unteransprüche.

### Beschreibung der Erfindung

Bei dem erfindungsgemäßen Verfahren zur Kohlenstoffdioxid-Verwertung mittels eines Elektrolysesystems wird ein Elektrolyt mit Kohlenstoffdioxid durch einen Kathodenraum an einer Kathode vorbeigeführt, wird Kohlenstoffdioxid an der Kathode reduziert und wenigstens eine Kohlenstoffwasserverbindung oder Kohlenstoffmonoxid als ein Elektrolyseprodukt sowie wenigstens ein Formiat als ein Elektrolysenebenprodukt erzeugt. Außerdem werden Elektrolysenebenprodukte mittels eines katalytischen Filtersystems aus dem Elektrolyt-Elektrolyse-Produktgemisch abgetrennt. Der erfindungsgemäße Einsatz des katalytischen Filtersystems für die unerwünschten Formiate im Elektrolysesystem, bzw. Elektrolytkreislauf, hat den Vorteil, dass eine Elektroden- oder eine Katalysatorverkokung durch Kohlenstoffablagerungen verhindert werden kann und dadurch insbesondere eine Deaktivierung einer Katalysatoroberfläche vermieden wird.

Unter Elektrolyseprodukten und Elektrolysenebenprodukten sind Substanzen zu verstehen, die mittels Elektrolyse erzeugt werden. Dabei sind Elektrolyseprodukte erwünschte Zielsubstanzen, Elektrolysenebenprodukte sind Substanzen, deren gleichzeitige Mit-Erzeugung im Wesentlichen unvermeidbar ist. Elektrolysenebenprodukte können tendenziell eher unerwünschte beziehungsweise häufig unnötige Substanzen sein. Unter Elektrolyseedukten sind Substanzen zu verstehen, die der Elektrolyse unterworfen werden.

Formiate sind die Salze der Ameisensäure und liegen je nach pH-Wert unterschiedlich dissoziiert vor. In saurem Milieu, unterhalb des pKs-Wertes von Ameisensäure liegt vornehmlich Formiat als Ameisensäure vor. In basischem Milieu liegt Formiat in Anionen und die entsprechende Anzahl an Kationen dissoziiert vor. Die erfindungsgemäß umsetzbaren Formiate sind typischerweise folgendermaßen darstellbar:

R⁺HCOO⁻

wobei R⁺ für ein Kation aus folgender Gruppe steht:
H+, Li+, Na+, K+, NH₄+, Cs+, Sr+, Ba+, Mn+, Cu+. Eine weitere bevorzugte Gruppe sind komplexe Kationen, wie sie beispielsweise in ionischen Flüssigkeiten vorkommen: ganz oder teilweise alkyliertes Ammonium, Phosphonium, Sulfonium, Piperidinium, Morpholinium, Pyridinium oder Imidazolinium oder deren Derivate.

Typischer Weise basiert das katalytische Filtersystem auf einem funktionalisierten Komplex oder Trägermaterial. Die eingesetzten funktionalisierten Komplexe oder Trägermaterialien haben die Eigenschaft, Formiate zu Wasserstoff und Kohlenstoffdioxid oder zu Wasser und Kohlenstoffmonoxid umzusetzen bzw. deren Umsetzung zu begünstigen. Dies geschieht nach einer der folgenden Reaktionsgleichungen:

HCOOH → H₂+CO₂ (1a)

HCOOH → H₂0+CO (1b)

Die funktionalisierten Komplexe sind dabei beispielsweise Übergangsmetallkomplexe, insbesondere mit einem Metall aus der achten Gruppe des Periodensystems oder Rhodium. Alternativ handelt es sich bei den funktionalisierten Komplexen um übergangsmetallfunktionalisierte Zeolithe, insbesondere y-Zeolithe, von denen besonders bevorzugt Na-Y-Zeolithe eingesetzt werden. In einer weiteren alternativen Ausführungsform der Erfindung werden als funktionalisierte Trägermaterialien Aktivkohle oder Tonerde eingesetzt. Tonerde ist das kubische γ-Al₂O₃, ein Ausgangsstoff zur Keramik- und Aluminium-Herstellung. Die Aktivkohle- oder Tonerde-Trägermaterialien werden bevorzugt übergangsmetallfunktionalisiert. Bevorzugt unter den funktionalisierten Komplexen werden 3-Aminopropyltrimethoxysilane-funktionalisierte Zeolithe eingesetzt. Erfindungsgemäß ist zur Kohlenstoffdioxid-Verwertung ein katalytisches Filtersystem zumindest teilweise auf der Kathodenoberfläche immobilisiert, welches nicht elektroreduktionskatalytisch aktiv ist. Dies stellt eine Variante dar, wie die Filtereinheit in die Elektrolysezelle integriert sein kann. Bevorzugt umfasst die Elektrolysezelle dazu eine Gasdiffusionselektrode.

In einer weiteren Ausführungsform des Verfahrens wird der Elektrolyt in einen geschlossenen Kreislauf geführt und eine Regenerierung des Filtersystems erfolgt thermisch.

Dazu werden bevorzugt besagte 3-Aminopropyltrimethoxysilanefunktionalisierte Na-Y-Zeolithe in einem im Elektrolytkreislauf eingebundenen Durchflussfilter eingesetzt. Dort agieren sie so zu sagen als Formiatfänger. Der Abfangmechanismus unterliegt der gezeigten Reaktion, bei der Amonium-Formiat gebildet wird:

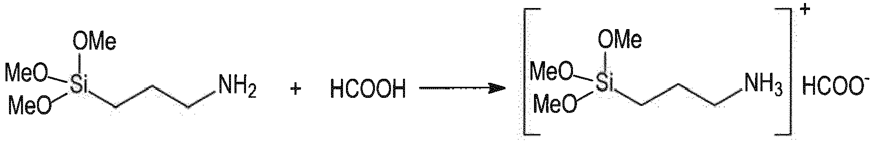

Die Filterregenerierung erfolgt in diesem Beispiel typischerweise zyklisch oder chargenweise durch thermische Behandlung.

Weiterhin wird die Verwendung von funktionalisierten Komplexen oder Trägermaterialien zur homogenen und heterogenen katalytischen Spaltung von Formiaten in Elektrolysesystemen zur Kohlenstoffdioxid-Verwertung angegeben. Funktionalisierte Trägermaterialien zur homogenen und heterogenen katalytischen Spaltung gemäß der Erfindung können beispielsweise sein:
- Aktivkohle,
- Tonerde.

Funktionalisierte Komplexe zur homogenen oder heterogenen katalytischen Spaltung von Formiaten in Elektrolysesystemen zur Kohlenstoffdioxid-Verwertung gemäß der Erfindung können beispielsweise sein:
- Übergangsmetallkomplexe, insbesondere mit einem Metall aus der 8. Gruppe des Periodensystems oder Rhodium, bevorzugt Eisen oder Ruthenium,
- Übergangsmetallfunktionalisierte Zeolithe, insbesondere Y-Zeolithe, darunter besonders geeignet Na-Y-Zeolithe,
- 3-Aminopropyltrimethoxysilan-funktionalisierte Zeolithe, insbesondere Y-Zeolithe.

Das erfindungsgemäße Elektrolysesystem zur Kohlenstoffdioxid-Verwertung umfasst eine Elektrolysezelle mit einer Anode in einem Anodenraum und mit einer Kathode in einem Kathodenraum sowie eine Filtereinheit, wobei der Kathodenraum ausgestaltet ist, ein Elektrolyseedukt, welches Kohlenstoffdioxid aufweist, aufzunehmen und an der Kathode K vorbeizuführen, wobei die Filtereinheit wenigstens ein katalytisches Filtersystem aufweist, mittels dem ein Formiat zu Wasserstoff und Kohlenstoffdioxid oder zu Wasser und Kohlenstoffmonoxid umgesetzt werden kann. Dabei ist das katalytische Filtersystem zumindest teilweise auf der Kathodenoberfläche immobilisiert und nicht elektroreduktionskatalytisch aktiv. Derartig umsetzbare Formiate sind beispielsweise des Typs R⁺HCOO⁻, wobei R⁺ für ein Kation aus folgender Gruppe steht: H+, Li+, Na+, K+, NH₄+, Cs+, Sr+, Ba+, Mn+, Cu+. Eine weitere bevorzugte Gruppe sind komplexe Kationen, wie sie beispielsweise in ionischen Flüssigkeiten vorkommen: ganz oder teilweise alkyliertes Ammonium, Phosphonium, Sulfonium, Piperidinium, Morpholinium, Pyridinium oder Imidazolinium oder deren Derivate.

Ein Vorteil dieses Elektrolysesystems mit Formiat-Filtereinheit besteht darin, dass es aufgrund der so vermiedenen Elektroden bzw. Katalysatorverkokung eine erhöhte Lebensdauer aufweist.

In einer typischen Ausführungsform des erfindungsgemäßen Elektrolysesystems weist das katalytische Filtersystem Übergangsmetallkomplexe auf, insbesondere mit einem Metall aus der 8. Gruppe des Periodensystems oder Rhodium. Alternativ dazu kann das katalytische Filtersystem auch übergangsmetallfunktionalisierte Zeolithe aufweisen, insbesondere Y-Zeolithe, besondere bevorzugt Na-Y-Zeolithe. In einer weiteren bevorzugten Ausführungsform des Elektrolysesystems weist das katalytische Filtersystem übergangsmetallfunktionalisierte Aktivkohle oder Tonerde als Trägermaterialien auf. Tonerde ist das kubische γ-Al₂O₃, ein Ausgangsstoff zur Keramik- und Aluminiumherstellung.

In einer weiteren bevorzugten Ausführungsform des Elektrolysesystems weist das katalytische Filtersystem 3-Aminopropyltrimethoxysilan-funktionalisierte Zeolithe auf, insbesondere Y-Zeolithe. In diesem Fall unterliegt der Abfangmechanismus oben gezeigter Reaktion (2) zur Bildung von Ammonium-Formiat und die Regenerierung des Filters kann in diesem Fall zyklisch oder chargenweise durch thermische Behandlung erfolgen.

Die Katalysatorvariante mit den übergangsmetallfunktionalisierten Komplexen oder Trägermaterialien bietet die Möglichkeit der katalytischen Spaltung von R⁺HCOO⁻ bei milden Reaktionsbedingungen nach einer der Reaktionen (1a) und (1b).

Milde Reaktionsbedingungen bedeutet, dass die Temperatur, bei der diese Reaktionen bevorzugt ablaufen, zwischen 0°C und 100°C liegt, vorzugsweise werden die Reaktionen bei Raumtemperatur vorgenommen. Der Druckbereich der Reaktionen liegt zwischen 1 bar und 100 bar, vorzugsweise bei 30 bar. Besonders die katalytische Spaltung zu Wasser und Kohlenstoffmonoxid (1b) hat den Vorteil, dass daraus sehr reines Kohlenstoffmonoxid gewonnen werden kann. Dementsprechend kann die Integration der Formiat-Filtereinheit in ein Elektrolysesystem zur Kohlenstoffdioxid-Verwertung auch vorteilhafterweise zur Kohlenstoffmonoxid-Herstellung eingesetzt.

In einer alternativen, nicht beanspruchten Ausgestaltung des Elektrolysesystems zur Kohlenstoffdioxid-Verwertung könnte die Filtereinheit einen von der Elektrolysezelle separaten Reaktionsraum umfassen. Diese Aufteilung kann von besonderem Vorteil für die thermische Regenerierungsbehandlung sein. Beispielsweise kann der separate Reaktionsraum als eine Art Durchflussfilter ausgestaltet sein, in dem die funktionalisierten Komplexe oder Trägermaterialien als Formiatfänger eingebunden sind.

Besonders bevorzugt ist die Ausführungsform des Elektrolysesystems mit einer Gasdiffusionselektrode als Formiat-Filter. Typischerweise weist diese dazu eine oberflächenmodifizierte Elektrode auf, auf der die funktionalisierten beispielsweise heterogenisierten Komplexe oder funktionalisierten Trägermaterialien immobilisiert sind.

Die beschriebenen Verfahren und Elektrolysesysteme ermöglichen eine kontinuierliche und effektive Abtrennung von Formiaten aus Elektrolytlösungen. Außerdem erhöht sich die Effizienz der Systeme, beispielsweise auch durch eine mögliche Wiedergewinnung der Ameisensäure aus den Formiaten. Alle beschriebenen Verfahren oder Systeme haben den Vorteil, dass zur Regeneration kein Elektrodenausbau vorgenommen werden muss. Die beschriebenen Formiat-Filter können jeweils in einem geschlossenen Elektrolytkreislauf eingesetzt werden. Von besonderem Vorteil ist der Einsatz einer Gasdiffusionselektrode als Formiat-Filter, deren Elektrodenoberfläche durch heterogenisierte Komplexverbindungen modifiziert ist. Die entsprechenden Modifikationen haben dabei typischerweise keinen negativen Einfluss auf den elektrokatalytischen Prozess.

Gemäß einer nicht beanspruchten Ausführungsform könnte sich der beschriebene Formiat-Filter beziehungsweise das beschriebene Filterverfahren derart zu Nutze gemacht werden, dass ein Formiat-Elektrolyseur zur Kohlenstoffdioxid-Verwertung und Kohlenstoffmonoxid-Herstellung realisiert wird. Basierend auf einem Elektrolysesystem wird darin Kohlenstoffdioxid an einer Kathode, welche einen Bleianteil aufweist, zu Formiat reduziert. An einer Bleielektrode kann dies mit einer nahezu 100 prozentigen Selektivität vorgenommen werden. Nach der Abtrennung der Gase aus dem Elektrolytkreislauf wird die oben beschriebene katalytische Spaltung der Formiate zu Wasser und Kohlenstoffmonoxid durchgeführt, typischerweise gemäß der Reaktionsgleichung 1b. Erzeugte Formiate sind in diesem Fall nicht länger als unerwünschte Elektrolysenebenprodukte anzusehen, sondern sind gezielt erzeugte Zwischenprodukte beziehungsweise Katalyseedukte.

Ausführungsformen der vorliegenden Erfindung werden in exemplarischer Weise mit Bezug auf die Figuren 1 bis 5 der angehängten Zeichnung beschrieben:
- Figur 1: zeigt ein Flussdiagramm für eine Elektrodenverkokung,
- Figur 2: zeigt in schematischer Darstellung ein Elektrolysesystem mit Formiat-Filter,
- Figur 3: zeigt in schematischer Darstellung ein Elektrolysesystem mit Gasdiffusionselektrode,
- Figur 4: zeigt schematisch einen Katholytkreislauf eines Kohlenstoffmonoxid-Elektrolyseurs und
- Figur 5: zeigt schematisch einen Katholytkreislauf eines Formiat-Elektrolyseurs.

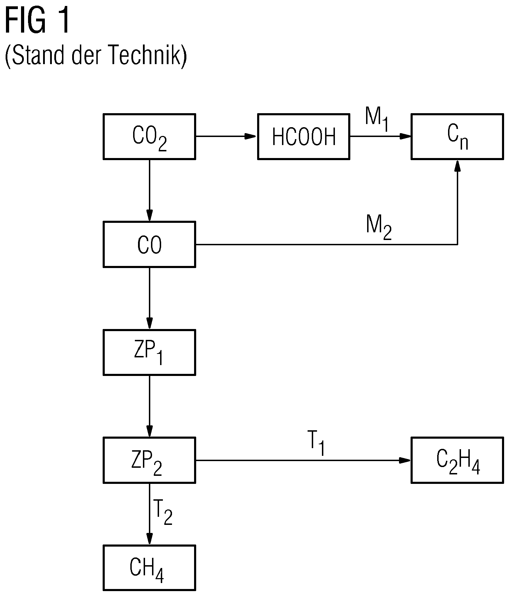

Figur 1 zeigt ein Flussdiagramm für eine Elektrodenverkokung am Beispiel der elektrokatalytischen Kohlenstoffdioxidreduktion, wie sie beispielsweise bei D. W. Dewulf, T. Jin, A. J. Bard, Journal of Electrochemical Society 1989, 136, 1686-1691 beschrieben ist. Kohlenstoffmonoxid CO ist nur ein Teilprodukt der elektrochemischen Reduktion von Kohlenstoffdioxid CO₂. Dieses leistet jedoch auch noch einen, zwar geringen, aber doch vorhandenen Beitrag M₂ zur Gesamtrate der Elektrodenverkokung. Wie bereits einführend erwähnt, sind besonders bevorzugte Produkte aus der Kohlenstoffdioxid-Verwertung Kohlenstoffmonoxid CO, Ethen C₂H₄ und Methan CH₄. Ethen C₂H₄ und Methan CH₄ entstehen über zwei weitere Zwischenschritte, beziehungsweise Zwischenprodukte ZP₁ und ZP₂, abhängig von den vorgegebenen Reaktionstemperaturen. Ein erstes Zwischenprodukt ZP₁ kann beispielsweise Cu-HCO umfassen, ein zweites Zwischenprodukt ZP₂ kann beispielsweises Cu-CH₂ umfassen. Bei hoher Temperatur T₂ um 321 K entsteht Ethen C₂H₄, bei niedrigerer Temperatur T₁ um 273 K wird Methan erzeugt. Der Hauptbeitrag M₁ zur Elektrodenverkokung entsteht über das unerwünschte Nebenprodukt Formiat HCOO⁻. Je mehr Kohlenstoff Cₙ sich auf der Elektrode und/oder dem Katalysator absetzt, wird die aktive Fläche reduziert und somit die Kohlenstoffdioxidreduktion vermindert, wobei in den meisten Fällen der elektrochemischen Reduktionsverfahren katalytisch aktive Elektroden eingesetzt werden.

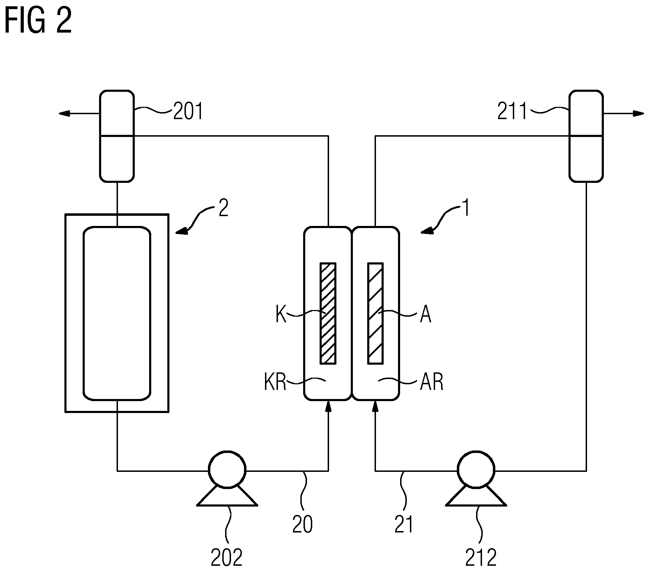

In Figur 2 ist schematisch ein beispielhafter Aufbau eines Elektrolysesystems gezeigt. Das System ist in diesem Fall so ausgestaltet, dass die Elektrolyse sowie die Filtereinheit kontinuierlich betrieben werden können. Dazu sind zwei Elektrolytkreisläufe mit je einer Umwälzpumpe gezeigt. Im Anolytkreislauf 21 wird mittels der Umwälzpumpe 212 der Elektrolyt durch einen Anodenraum AR an einer Anode A vorbeigeführt. Die Durchflussrichtung ist durch einen Pfeil angezeigt. In Durchflussrichtung anschließend an die Elektrolysezelle 1 befindet sich ein Phasenabscheider 211, über den z.B. das an der Anode A beispielsweise entstehende Sauerstoffgas O₂ ausgeleitet werden kann. Der Katholytkreislauf 20 weist ebenso eine Umwälzpumpe 202 auf, welche den Elektrolyten und Elektrolyseedukte durch den Kathodenraum KR an der Kathode K vorbeiführt. In Durchflussrichtung, wiederum durch einen Pfeil angezeigt, nach dem Kathodenraum KR befindet sich wiederum ein Phasenabscheider 201, über welchen Gase aus dem Kreislauf 20 ausgeleitet werden können. Bei den aus dem Phasenabscheider 201 ausgeleiteten Gasen handelt es sich beispielsweise um überschüssiges Kohlenstoffdioxid CO₂, welches nicht umgesetzt wurde. Zusätzlich umfasst der Katholytkreislauf 20 eine Katalysator- bzw. Filtereinheit 2.

Diese Aufteilung kann von besonderem Vorteil für die thermische Regenerierungsbehandlung sein. Beispielsweise kann der separate Reaktionsraum 2 als eine Art Durchflussfilter ausgestaltet sein, in dem die funktionalisierten Komplexe oder Trägermaterialien als Formiatfänger eingebunden sind. Die integrierte Filtereinheit 2 in den Katholytkreislauf 20 vermeidet, dass sich Formiate im Kathodenkreislauf zunehmend anreichern und damit die katalytischen Zentren durch eine Graphitablagerung blockieren.

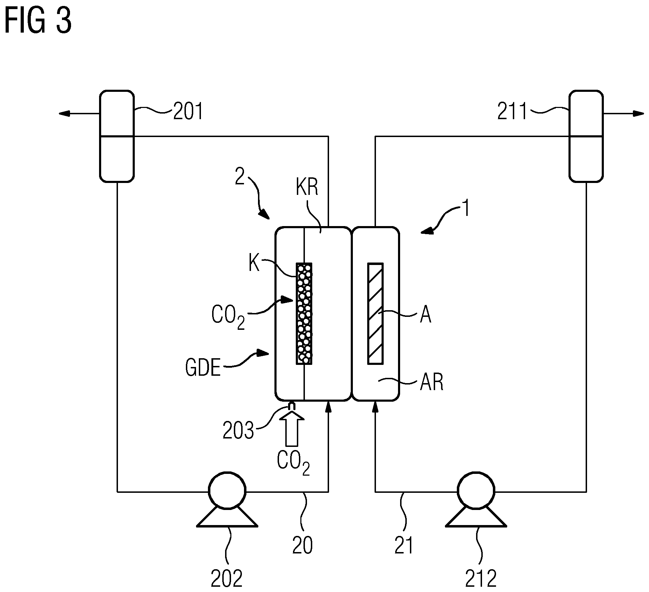

Alternativ zu Figur 2 könnte die Katalysator- bzw. Filtereinheit 2 auch in den Kathodenraum KR integriert sein. Dies könnte beispielsweise in Form einer Gasdiffusionselektrode GDE realisiert werden. Das in Figur 3 gezeigte Elektrolysesystem unterscheidet sich von dem in Figur 2 durch den Aufbau der Elektrolysezelle 1: Anstelle eines Zwei-Kammer-Aufbaus weißt diese nun eine Gasdiffusionselektrode GDE auf. Zur Einbringung des Kohlenstoffdioxids CO₂ in den Katholytkreislauf 20 umfasst die Gasdiffusionselektrode GDE einen Kohlenstoffdioxideinlass 203, die Kathode K ist für das Kohlestoffdioxid CO₂ gasdurchlässig. Eine Gasdiffusionselektrode GDE ist dadurch charakterisiert, dass eine flüssige Komponente, z.B. ein Elektrolyt, sowie eine gasförmige Komponente, z.B. ein Elektrolyseedukt, in einem Porensystem der Elektrode, z.B. der Kathode K, in Kontakt miteinander gebracht werden können. Das Porensystem der Elektrode ist dabei so ausgeführt, dass die flüssige sowie die gasförmige Phase gleichermaßen in das Porensystem eindringen können und darin gleichzeitig vorliegen können. Typischerweise ist dazu ein Reaktionskatalysator porös ausgeführt und übernimmt die Elektrodenfunktion, oder eine poröse Elektrode weist katalytisch wirkende Komponenten auf. Im vorliegenden Beispiel kann die Gasdiffusionselektrode GDE die Formiat-Fänger beinhalten, also die funktionalisierten, beispielsweise heterogenisierten, Komplexe oder funktionalisierten Trägermaterialien. Diese können auf einer oberflächenmodifizierten Elektrode oder anderen Innenflächen des Kathodenraums KR immobilisiert sein.

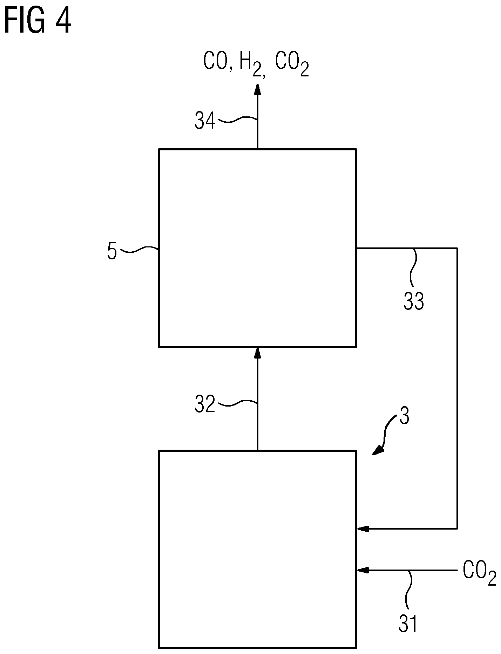

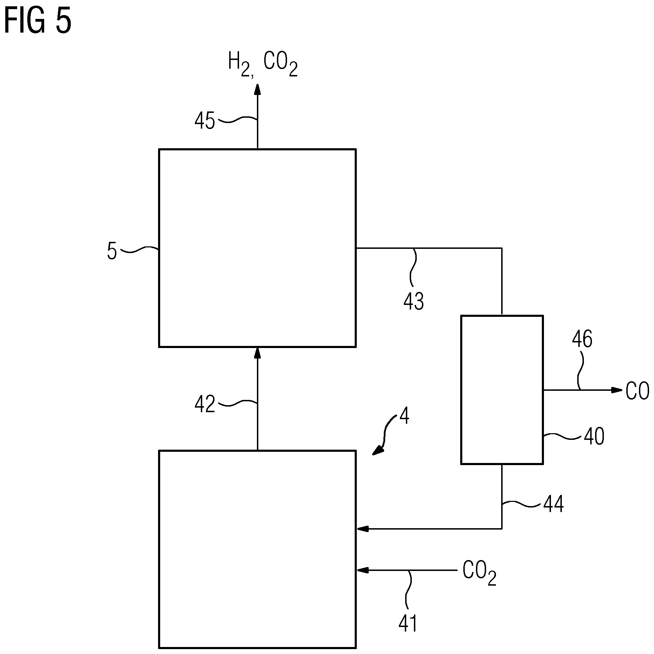

In den Figuren 4 und 5 sind exemplarisch die Katholytkreisläufe eines Kohlenstoffmonoxid-Elektrolyseurs und eines Formiat-Elektrolyseurs dargestellt.

Figur 4 verdeutlicht noch einmal die Problematik der Gewinnung von reinen Produkten aus einem Kohlenstoffdioxid-Verwertungsprozess mittels Elektrolyse: Durch die Selektivität der verwendeten Elektroden können die Reduktionsprodukte bis zu einem gewissen Prozentanteil vorbestimmt werden. Die Gewinnung von reinem Kohlenstoffmonoxid CO aus einer Kohlenstoffdioxidreduktion ist jedoch nicht möglich. Wie in Figur 4 gezeigt, verlassen die Elektrolysezelle 3 stets mehrere Produkte: Kohlenstoffmonoxid CO, Kohlenstoffdioxid CO₂ und Wasserstoff H₂. Nach der Gasabtrennung 5 können diese aus dem System als Gasgemisch 34 ausgeleitet werden, während der Elektrolyt beispielsweise über die Leitung 33 in einem Kreislauf wieder in die Elektrolysezelle 3 eingeführt. Des Weiteren ist in der Figur 4 der Kohlenstoffdioxidzulauf 31 gezeigt. Beispielsweise kann das Kohlenstoffdioxid CO₂ bei Verwendung einer Gasdiffusionselektrode an dieser Stelle in das System eingebracht werden oder über ein separates Kohlenstoffdioxidreservoir in den Elektrolytkreislauf eingebracht werden. Ganz im Gegensatz zu den Wasserstoff-Elektrolyseuren ist bei der Kohlenstoffdioxidelektrolyse die Trennung zwischen Produkten, also dem Kohlenstoffmonoxid CO und den Edukten, eben dem Kohlenstoffdioxid CO₂ offensichtlich deutlich aufwendiger, da sowohl Produkte als auch Edukte in Gasform vorliegen.

Macht man sich die katalytische Zersetzungsreaktion von HCOOH beziehungsweise HCOO⁻ zu H₂0 und CO (1c) zunutze, kann aus der Katalysator- und Filtereinheit 2 neben deren Filterwirkung auch sehr reines Kohlenstoffmonoxid CO gewonnen werden: In dem erfindungsgemäßen Formiat-Elektrolyseur macht man sich die nahezu mit 100%iger Selektivität aus Kohlenstoffdioxid CO₂ erzeugbaren Formiate R⁺HCOO⁻ entsprechend zunutze. Diese sind in diesem Verfahren bzw. dieser Vorrichtung zur Kohlenstoffdioxid-Verwertung nicht länger unerwünschte Reaktionsnebenprodukte, sondern gezielt erzeugte Zwischenprodukte auf dem Weg zur Kohlenstoffmonoxid-Herstellung. In dem in Figur 5 gezeigten Formiat-Elektrolyseur wird also in der Elektrolysezelle 4 nicht Kohlenstoffdioxid CO₂ zu Kohlenstoffmonoxid CO reduziert, sondern das Kohlenstoffdioxid CO₂ wird an einer Bleielektrode zu Formiat R⁺HCOO⁻ reduziert. Dieses ist im Elektrolyten gelöst und durch eine Gasabtrennung kann nun dieses Zwischenprodukt von dem nicht umgesetzten gasförmigen Edukt, also dem Kohlenstoffdioxid CO₂ abgetrennt werden. Das heißt z.B. weiterhin, dass hier keine teure Silberelektrode verwendet werden muss.

Der formiathaltige Elektrolyt wird dann über die Katalysator- bzw. Filtereinheit 40 weitergeleitet. In dieser Filtereinheit 40 erfolgt die Formiatspaltungsreaktion: Das Formiat R⁺HCOO⁻ wird zu Kohlenstoffmonoxid CO umgewandelt, vorzugsweise gemäß der Reaktion 1b. Im Gegensatz zur Standardelektrolyse von Kohlenstoffdioxid CO₂, bei der Kohlenstoffmonoxid CO zusammen mit Wasserstoff H₂ und Kohlenstoffdioxid CO₂ Verunreinigungen erzeugbar ist, welche sich auf einen Anteil von bis zu mehreren Prozent belaufen können, kann mittels dem Formiate-Elektrolyseur sehr reines Kohlenstoffmonoxid CO hergestellt werden. Die Verunreinigungen liegen dann nur noch im Promillebereich. Von besonderem Vorteil ist weiterhin, dass der Formiate-Elektrolyseur keine zusätzliche aufwendige Gasabtrennung benötigt. So kann wasserstofffreies Kohlenstoffmonoxid CO hergestellt werden, was aktuell eine sehr teure Ressource der chemischen Industrie ist und gleichzeitig ein effektives, langlebiges Elektrolysesystem zur Kohlenstoffdioxid-Verwertung zur Verfügung gestellt werden.

## Patentansprüche

1. Verfahren zur Kohlenstoffdioxid-Verwertung mittels eines Elektrolysesystems,
- bei dem ein Elektrolyt und Kohlenstoffdioxid (CO₂) durch einen Kathodenraum (KR) an einer Kathode (K) vorbeigeführt werden,
- bei dem die Kathode (K) ein Elektroden- und/oder Katalysatormaterial aufweist, womit Kohlenstoffdioxid (CO₂) an der Kathode (K) reduziert und wenigstens eine Kohlenwasserstoffverbindung oder Kohlenstoffmonoxid (CO) als Elektrolyseprodukt sowie wenigstens ein Formiat als Elektrolysenebenprodukt erzeugt wird und
- bei dem Elektrolysenebenprodukte mittels eines zumindest teilweise auf der Kathodenoberfläche immobilisierten katalytischen Filtersystems, welches nicht elektroreduktionskatalytisch aktiv ist, aus dem Elektrolyt-Elektrolyseprodukt-Gemisch abgetrennt werden, wobei das katalytische Filtersystem einen funktionalisierten Komplex oder ein funktionalisiertes Trägermaterial aufweist welches eine Spaltungsreaktion von Formiaten zu Wasserstoff (H₂) und Kohlenstoffdioxid (CO₂) oder zu Wasser (H₂O) und Kohlenstoffmonoxid (CO) katalysiert.

2. Verfahren nach Anspruch 1 bei dem der Elektrolyt in einem geschlossenen Kreislauf geführt wird und eine Regenerierung des Filtersystems thermisch erfolgt.

3. Elektrolysesystem zur Kohlenstoffdioxid-Verwertung, umfassend eine Elektrolysezelle (4) mit einer Anode (A) in einem Anodenraum (AR) und mit einer Kathode (K) in einem Kathodenraum (KR), und eine Filtereinheit (40),
- wobei der Kathodenraum (KR) ein Elektrolyseedukt umfasst, welches Kohlenstoffdioxid aufweist, und ausgestaltet ist das Elektrolyseedukt an der Kathode vorbei zu führen,
- wobei die Kathode (K) ein Elektroden- und/oder Katalysatormaterial aufweist, mittels welchem Kohlenstoffdioxid (CO₂) zu wenigstens einer Kohlenwasserstoffverbindung oder zu Kohlenstoffmonoxid (CO) als Elektrolyseprodukt sowie zu wenigstens einem Formiat als Elektrolysenebenprodukt reduzierbar ist,
- wobei die Filtereinheit (40) wenigstens ein katalytisches Filtersystem aufweist, mittels dem ein Formiat zu Wasserstoff (H₂) und Kohlenstoffdioxid (CO₂) oder zu Wasser (H₂O) und Kohlenstoffmonoxid (CO) umgesetzt werden kann
- und wobei das katalytische Filtersystem zumindest teilweise auf der Kathodenoberfläche immobilisiert und nicht elektroreduktionskatalytisch aktiv ist.

4. Elektrolysesystem zur Kohlenstoffdioxid-Verwertung nach Anspruch 3, wobei das umsetzbare Formiat des Typs R⁺HCOO⁻ ist und R⁺ für ein Kation aus folgender Gruppe steht: H⁺, Li⁺, Na⁺, K⁺, NH4⁺, Cs⁺, Sr⁺, Ba⁺, Mn⁺, Cu⁺.

5. Elektrolysesystem zur Kohlenstoffdioxid-Verwertung nach Anspruch 3 oder 4, wobei das katalytische Filtersystem auf Übergangsmetallkomplexen basiert, insbesondere mit einem Metall aus der achten Gruppe des Periodensystems oder Rhodium.

6. Elektrolysesystem zur Kohlenstoffdioxid-Verwertung nach einem der Ansprüche 3 oder 4, wobei das katalytische Filtersystem auf übergangsmetallfunktionalisierten Zeolithen basiert, insbesondere Y-Zeolithen.

7. Elektrolysesystem zur Kohlenstoffdioxid-Verwertung nach einem der Ansprüche 3 oder 4, wobei das katalytische Filtersystem auf übergangsmetallfunktionalisierter Aktivkohle oder Tonerde als Trägermaterialien basiert.

8. Elektrolysesystem zur Kohlenstoffdioxid-Verwertung nach einem der Ansprüche 3 oder 4, wobei das katalytische Filtersystem auf 3-Aminopropyltrimethoxysilane-funktionalisierten Zeolithen basiert, insbesondere Y-Zeolithen.

9. Elektrolysesystem zur Kohlenstoffdioxid-Verwertung nach einem der Ansprüche 3 bis 8, mit einer Gasdiffusionselektrode als Formiate-Filter.

## Claims

1. Method for carbon dioxide utilization by means of an electrolysis system,
- wherein an electrolyte and carbon dioxide (CO₂) are passed in front of a cathode (K) through a cathode chamber (KR),
- wherein the cathode (K) comprises an electrode and/or catalyst material with which carbon dioxide (CO₂) is reduced at the cathode (K) and at least one hydrocarbon compound or carbon monoxide (CO) is generated as electrolysis product and also at least one formate is generated as electrolysis byproduct, and
- wherein electrolysis byproducts are removed from the electrolyte/electrolysis product mixture by means of a catalytic filter system which is immobilized at least partly on the cathode surface and is not electroreductively catalytically active, said catalytic filter system comprising a functionalized complex or a functionalized support material which catalyzes a cleavage reaction of formates to hydrogen (H₂) and carbon dioxide (CO₂) or to water (H₂O) and carbon monoxide (CO).

2. Method according to Claim 1, wherein the electrolyte is guided in a closed circuit and the filter system is regenerated thermally.

3. Electrolysis system for carbon dioxide utilization, comprising an electrolysis cell (4) having an anode (A) in an anode chamber (AR) and having a cathode (K) in a cathode chamber (KR), and a filter unit (40),
- where the cathode chamber (KR) comprises an electrolysis reactant comprising carbon dioxide and is designed to pass said electrolysis reactant in front of the cathode,
- where the cathode (K) comprises an electrode and/or catalyst material by means of which carbon dioxide (CO₂) can be reduced to at least one hydrocarbon compound or to carbon monoxide (CO) as electrolysis product and also to at least one formate as electrolysis byproduct,
- where the filter unit (40) comprises at least one catalytic filter system by means of which a formate can be converted to hydrogen (H₂) and carbon dioxide (CO₂) or to water (H₂O) and carbon monoxide (CO),
- and where the catalytic filter system is immobilized at least partly on the cathode surfce and is not electroreductively catalytically active.

4. Electrolysis system for carbon dioxide utilization according to Claim 3, where the reactable formate is of the type R⁺HCOO⁻ and R⁺ is a cation from the following group: H⁺, Li⁺, Na⁺, K⁺, NH4⁺, Cs⁺, Sr⁺, Ba⁺, Mn⁺, Cu⁺.

5. Electrolysis system for carbon dioxide utilization according to Claim 3 or 4, where the catalytic filter system is based on transition metal complexes, more particularly with a metal from the eighth group of the periodic table or rhodium.

6. Electrolysis system for carbon dioxide utilization according to either of Claims 3 and 4, where the catalytic filter system is based on transition-metal-functionalized zeolites, more particularly Y zeolites.

7. Electrolysis system for carbon dioxide utilization according to either of Claims 3 and 4, where the catalytic filter system is based on transition-metal-functionalized activated carbon or alumina as support materials.

8. Electrolysis system for carbon dioxide utilization according to either of Claims 3 and 4, where the catalytic filter system is based on 3-aminopropyltrimethoxysilane-functionalized zeolites, more particularly Y zeolites.

9. Electrolysis system for carbon dioxide utilization according to any of Claims 3 to 8, having a gas diffusion electrode as formate filter.

## Revendications

1. Procédé de valorisation du dioxyde de carbone au moyen d'un système d'électrolyse,
- dans lequel on fait passer, sur une cathode (K), un électrolyte et du dioxyde de carbone (CO₂) dans un compartiment (KR) cathodique,
- dans lequel la cathode (K) a un matériau d'électrode et/ou de catalyseur, grâce à quoi on réduit du dioxyde de carbone (CO₂) à la cathode (K) et on produit au moins un composé hydrocarboné ou du monoxyde de carbone (CO) comme produit d'électrolyse, ainsi qu'au moins un formiate comme sous-produit d'électrolyse et
- dans lequel on sépare du mélange électrolyte-produit d'électrolyse des sous-produits de l'électrolyse au moyen d'un système de filtre catalytique, qui est immobilisé, au moins en partie, sur la surface de la cathode et qui n'est pas actif en catalyse d'électro-réduction, le système de filtre catalytique ayant un complexe fonctionnalisé ou une matière support fonctionnalisée, qui catalyse une réaction de dissociation de formiates en hydrogène (H₂) et en dioxyde de carbone (CO₂) ou en eau (H₂O) et en monoxyde de carbone (CO).

2. Procédé suivant la revendication 1, dans lequel on fait passer l'électrolyte dans un circuit fermé et on effectue thermiquement une régénération du système de filtre.

3. Système d'électrolyse pour la valorisation du dioxyde de carbone, comprenant une cellule (4) d'électrolyse, ayant une anode (A) dans un compartiment (AR) anodique et une cathode (K) dans un compartiment (KR) cathodique, et une unité (40) de filtre,
- dans lequel le compartiment (KR) cathodique comprend un éduit d'électrolyse, qui a du dioxyde de carbone, et est conformé pour faire passer l'éduit d'électrolyse devant la cathode,
- dans lequel la cathode (K) a une matière d'électrode et/ou de catalyseur au moyen de laquelle le dioxyde de carbone (CO₂) peut être réduit en au moins un composé hydrocarboné ou en monoxyde de carbone (CO) comme produit de l'électrolyse, ainsi qu'en au moins un formiate comme sous-produit de l'électrolyse,
- dans lequel l'unité (40) de filtre a au moins un système de filtre catalytique, au moyen duquel un formiate peut être transformé en hydrogène (H₂) et dioxyde de carbone (CO₂) ou en eau (H₂O) et en monoxyde de carbone (CO)
- et dans lequel le système de filtre catalytique est immobilisé, au moins en partie, à la surface de la cathode et n'est pas actif en catalyse d'électro-réduction.

4. Système d'électrolyse pour la valorisation du dioxyde de carbone suivant la revendication 3, dans lequel le formiate, pouvant être dissocié est du type R⁺HCOO⁻ et R⁺ est un cation du groupe suivant : H⁺, Li⁺, Na⁺, K⁺, NH4⁺, Cs⁺, Sr⁺, Ba⁺, Mn⁺, Cu⁺.

5. Système d'électrolyse pour la valorisation du dioxyde de carbone suivant la revendication 3 ou 4, **caractérisé en ce que** le système de filtre catalytique repose sur des complexes de métal de transition, notamment avec un métal du huitième groupe de la classification périodique ou le rhodium.

6. Système d'électrolyse pour la valorisation du dioxyde de carbone suivant l'une des revendication 3 ou 4, dans lequel le système de filtre catalytique repose sur des zéolithes fonctionnalisées par du métal de transition, notamment des zéolithes Y.

7. Système d'électrolyse pour la valorisation du dioxyde de carbone suivant l'une des revendications 3 ou 4, dans lequel le système de filtre catalytique repose sur du charbon actif fonctionnalisé par un métal de transition ou de l'alumine comme matériaux de support.

8. Système d'électrolyse pour la valorisation du dioxyde de carbone suivant l'une des revendications 3 ou 4, dans lequel le système de filtre catalytique repose sur des zéolithes fonctionnalisées par du 3-aminopropyltriméthoxysilane, notamment des zéolithes Y.

9. Système d'électrolyse pour la valorisation du dioxyde de carbone suivant l'une des revendications 3 à 8, comprenant une électrode à diffusion de gaz comme filtre de formiates.
